(19) Europäisches Patentamt
European Patent Office
Office européen des brevets

(11) Veröffentlichungsnummer: **0 224 057**
**B1**

(12) # EUROPÄISCHE PATENTSCHRIFT

(45) Veröffentlichungstag der Patentschrift: **23.08.89**

(51) Int. Cl.⁴: **C07D 261/04**

(21) Anmeldenummer: **86114930.0**

(22) Anmeldetag: **27.10.86**

(54) **Verfahren zur Herstellung von Isoxazolidin-3-onen.**

(30) Priorität: **16.11.85 DE 3540770**

(43) Veröffentlichungstag der Anmeldung: **03.06.87 Patentblatt 87/23**

(45) Bekanntmachung des Hinweises auf die Patenterteilung: **23.08.89 Patentblatt 89/34**

(84) Benannte Vertragsstaaten: **CH DE FR GB LI**

(56) Entgegenhaltungen:
**FR-A- 2 483 406**

**BULLETIN DE LA SOCIETE CHIMIQUE DE FRANCE, 2ème partie intitulée: "Chimie moléculaire", Nr. 9-10, September-Oktober 1976, Seiten 1445-1606; J.-L. OLIVE et al.: "Action de l'hydroxyurée sur les esters alpha-éthyléniques. Synthèse et caractérisation d'isoxazolidinones-3" 000**

(73) Patentinhaber: **BASF Aktiengesellschaft, Carl-Bosch-Strasse 38, D-6700 Ludwigshafen(DE)**

(72) Erfinder: **Westphalen, Karl-Otto, Dr., Mausbergweg 58, D-6720 Speyer(DE)**
Erfinder: **Hamprecht, Gerhard, Dr., Rote-Turm-Strasse 28, D-6940 Weinheim(DE)**
Erfinder: **Hettinger, Peter, Dr., Schloss-Strasse 3, D-6803 Edingen-Neckarhausen(DE)**

**EP 0 224 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von substituierten Isoxazolidin-3-onen oder von deren Salzen durch Umsetzung von Hydroxylamin mit substituierten 3-Chlor- oder Brompropionsäureestern in Gegenwart von Base.

In der DE-OS 31 21 704 wird ein zweistufiges Verfahren zur Herstellung von Isoxazolidin-3-onen durch Umsetzung von Hydroxylaminen mit 3-halogensubstituierten Carbonsäurechloriden in Gegenwart einer Base beschrieben. Die im ersten Reaktionsschritt gebildete Hydroxamsäure wird nach Isolierung und Reinigung in einem zweiten Reaktionsschritt zum Heterocyclus cyclisiert. So liefert die Umsetzung von Chlorpivalinsäurechlorid mit 2 Äquivalenten Hydroxylaminhydrochlorid in Wasser in Gegenwart von Natronlauge die Hydroxamsäure in einer Ausbeute von nur 40 %. Dieses Zwischenprodukt wird dann in Methanol als Lösungsmittel in Gegenwart von überschüssigem KOH zum 4,4-Dimethyl-isoxazolin-3-on umgesetzt (Beispiel 30). Die Gesamtausbeute bezogen auf Chlorpivalinsäurechlorid beträgt 38 %. Es wird erwähnt, daß anstelle von Säurechloriden auch Methyl- oder Ethylester umgesetzt werden können. In diesem Fall sollten die Ausbeuten aufgrund der geringen Reaktivität der Esterfunktion noch niedriger ausfallen.

Nachteilig an dem beschriebenen Verfahren sind die aufwendige zweistufige Verfahrensweise und die niedrigen Ausbeuten. Außerdem müssen je Mol Säurechlorid 2 mol des toxischen Hydroxylamins verwendet werden, wodurch pro Mol Säurechlorid 1 mol Hydroxylamin in die wäßrige Phase gelangt, aus der es aufgrund seiner hohen Wasserlöslichkeit ohne großen Aufwand nicht zurückgewonnen werden kann. Auch bei der Herstellung des Chlorpivalinsäurechlorids durch Umsetzung der Säure mit Thionylchlorid entstehen die die Umwelt belastenden Stoffe Schwefeldioxid und Chlorwasserstoff, die z.B. durch Waschtürme entsorgt werden müssen.

Der Erfindung lag nun die Aufgabe zugrunde, ein umweltfreundliches Verfahren zur Herstellung von Isoxazolin-3-onen zu finden, das sich durch einfache und wirtschaftliche Arbeitsweise auszeichnet.

Demgemäß wurde ein Verfahren zur Herstellung von Isoxazolidin-3-onen der Formel I

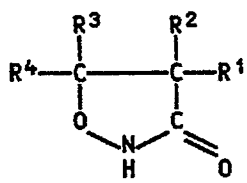

I,

in der $R^1$ und $R^2$ verzweigte oder unverzweigte Alkylreste mit 1 bis 8 Kohlenstoffatomen bedeuten, die unter den Reaktionsbedingungen inerte Substituenten tragen können und $R^3$ und $R^4$ Wasserstoff, Chlor, Brom oder einen aliphatischen Rest darstellen, oder von deren Salzen, durch Umsetzung von Hydroxylamin oder dessen Salzen mit einem Ester der Formel II

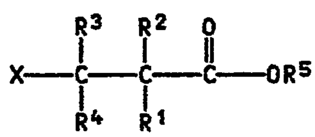

II,

in der X für Chlor oder Brom steht und $R^5$ einen aliphatischen, cycloaliphatischen, araliphatischen oder aromatischen Rest bedeutet, in Gegenwart einer Base und gegebenenfalls Freisetzen der Isoxazolidin-3-one aus deren Salzen durch Ansäuern des Reaktionsgemisches gefunden, das sich besonders vorteilhaft durchführen läßt, wenn man die Umsetzung in einem inerten organischen Lösungsmittel oder einem wäßrig organischen Lösungsmittelgemisch ohne Isolierung von Zwischenstufen durchführt.

Bei Verwendung von Hydroxylaminhydrochlorid und 3-Chlorpivalinsäuremethylester als Ausgangsstoffe und Natriummethylat als Base wird die Umsetzung durch folgende Reaktionsgleichung beschrieben:

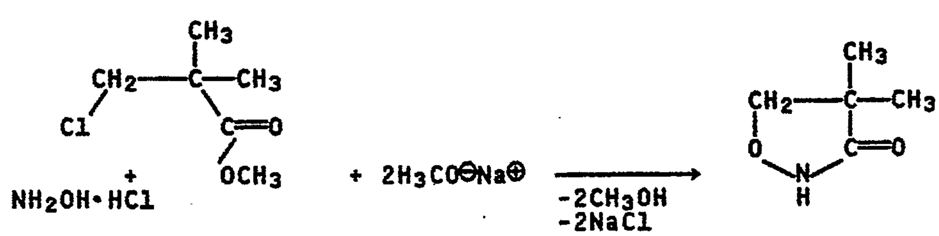


Anstelle des freien Hydroxylamins lassen sich vorteilhaft dessen Salze z.B. das Hydrochlorid, Sulfat, Hydrogensulfat, Carbonat oder Hydrogencarbonat verwenden.

Ausgangsstoffe der Formel II sind solche, in denen X für Chlor oder Brom steht und $R_1$ und $R^2$ verzweigte oder unverzweigte Alkylreste mit 1 bis 8, insbesondere 1 bis 4 Kohlenstoffatomen darstellen, die gegebenenfalls durch unter den Reaktionsbedingungen inerte Substituenten wie Halogen, z.B. Fluor, Chlor oder Brom, Cyanid oder Alkoxy- bzw. Thioalkylreste mit 1 bis 4 Kohlenstoffatomen substituiert sein können.

Beispielsweise stehen $R^1$ oder $R^2$ für eine Methyl-, Chlormethyl-, Ethyl-, Propyl-, iso-Propyl-, Butyl, iso-Butyl- oder tert.-Butylgruppe.

Als Reste $R^3$ und $R^4$ kommen Wasserstoff, Chlor, Brom oder aliphatische Reste in Betracht. Aliphatische Reste sind z.B. Alkylreste mit 1 bis 8 C-Atomen, die durch Chlor oder Brom substituiert sein können, sowie Alkoxy- oder Thioalkylreste mit insbesondere 1 bis 4 C-Atomen. Beispielsweise seien die Methyl-, Chlormethyl-, Dichlormethyl-, Ethyl-, 2-Bromethyl-, Propyl-, iso-Propyl-, Butyl-, Methoxy-, Ethoxy-, Methylthio- und die Ethylthiogruppe genannt. Vorzugsweise stehen $R^3$ und/oder $R^4$ für Wasserstoff, Chlor oder Alkylgruppen mit 1 bis 4 C-Atomen.

Bezüglich des organischen Restes $R^5$ bestehen praktisch keine Beschränkungen; er kann einen aliphatischen, cycloaliphatischen, araliphatischen oder aromatischen Rest darstellen, z.B. einen Alkylrest mit 1 bis 15, insbesondere 1 bis 8 C-Atomen, einen Cycloalkylrest mit 4 bis 6 C-Atomen, der ein weiteres Heteroatom wie Stickstoff oder Sauerstoff enthalten kann, einen Aralkylrest mit 7 bis 12 C-Atomen oder einen Arylrest. Alkyl- oder Cycloalkylreste sind beispielsweise der Methyl-, Ethyl-, Propyl-, iso-Propyl-, n- und iso-Butyl-, Pentyl-, Hexyl-, Cyclopentyl- oder Cyclohexylrest.

Die Ausgangsstoffe II sind aus den entsprechenden Carbonsäuren in an sich bekannter Weise gut zugänglich.

Die Umsetzung der Ausgangsstoffe erfolgt in einem inerten Lösungsmittel in Gegenwart eines Säureakzeptors. Als Säureakzeptoren können Zink-, Alkalimetall- und Erdalkalimetallverbindungen, zweckmäßig Carbonate, Bicarbonate, Oxide, Azetate, Formiate und vorteilhaft Hydroxide oder Alkoholate insbesondere von Alkanolen mit 1 bis 4 C-Atomen, sowie entsprechende Gemische verwendet werden. Es kommen z.B. als basische Verbindungen in Betracht: Kaliumhydroxid, Natriumhydroxid, Natriumcarbonat, Kaliumcarbonat, Lithiumhydroxid, Lithiumcarbonat; Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkhydrogencarbonat, Zinkacetat, Natriumformiat und Natriumacetat, Natrium- oder Kaliummethylat, -ethylat, -propylat oder -butylat.

Als Lösungsmittel können inerte, organische Lösungsmittel oder wäßrigorganische Lösungsmittelgemische, wobei der Anteil des Wassers an der gesamten Lösungsmittelmenge 10 bis 50 Gew.%, vorzugsweise 20 bis 40 Gew.% beträgt, verwendet werden. Geeignete Lösungsmittel sind beispielsweise chlorierte Kohlenwasserstoffe wie Methylenchlorid, Chloroform, 1,1- und 1,2-Dichlorethan, Dichlorbutan, mono-Chlor- oder Dichlorbenzol, Ether wie Diethylether, Methyl-tert.-butylether, Diisopropylether oder Tetrahydrofuran sowie aliphatische, cycloaliphatische oder aromatische Kohlenwasserstoffe wie Pentan, Hexan, Cyclohexan, Petrolether, Toluol oder Xylol. Besonders vorteilhaft lassen sich Alkohole wie Methanol, Ethanol, Propanol, Isopropanol, Butanol, Hexanol oder Cyclohexanol als Lösungsmittel verwenden. Zweckmäßig wird das Lösungsmittel oder das wäßrigorganische Lösungsmittelgemisch in einer Menge von 100 bis 2000 Gew.%, vorzugsweise 200 bis 1000 Gew.%, bezogen auf den Ausgangsstoff II, verwendet.

Die Ausgangsstoffe können in stöchiometrischer Menge oder im Überschuß der einen Komponente zur anderen eingesetzt werden. Vorzugsweise wird die Umsetzung in Gegenwart möglichst geringer Mengen an Hydroxylamin durchgeführt. So sind im allgemeinen je Mol Ausgangsstoff II 0,8 bis 1,5, insbesondere 1,0 bis 1,1 mol Hydroxylamin ausreichend. Die Menge an zugesetzter Base kann 2 bis 6 mol, vorzugsweise 2 bis 4 mol pro Mol Ausgangsstoff II betragen.

Nach dem erfindungsgemäßen Verfahren erfolgt die Umsetzung zu den Isoxazolidin-3-onen ohne Isolierung von Zwischenstufen. Die Reaktionstemperatur ist nicht besonders kritisch und kann -10 bis 150°C betragen. Zweckmäßigerweise wird die Umsetzung bei Temperaturen von 10 bis 80, insbesondere 20 bis 40°C vorgenommen.

Die Durchführung des Verfahrens kann kontinuierlich oder diskontinuierlich bei Über- oder Unterdruck, vorzugsweise bei Normaldruck nach den dafür üblichen Techniken erfolgen.

Nach der Umsetzung liegen die Isoxazolidin-3-one in Form ihrer Salze vor und können aus diesen durch Ansäuern des Reaktionsgemisches mit Mineralsäuren wie HCl oder $H_2SO_4$ oder organischen Säuren wie Ameisen-oder Essigsäure freigesetzt werden. Die Isolierung der Produkte erfolgt in an sich bekannter Weise, indem man z.B. das Lösungsmittel entfernt, den Rückstand mit Wasser aufnimmt und mit einem organischen Lösungsmittel extrahiert. Zur Reinigung können die Produkte z.B. destilliert oder umkristallisiert werden.

Die nach dem erfindungsgemäßen Verfahren erhältlichen Verbindungen dienen hauptsächlich als Zwischenprodukte für die Herstellung von Herbiziden, wie sie beispielsweise in der DE-OS 31 21 704 beschrieben sind.

Beispiel

Herstellung von 4,4-Dimethylisoxazolidin-3-on

92,4 g (1,328 mol) Hydroxylaminhydrochlorid wurden in 800 ml Methanol vorgelegt, mit 95,2 g (1,76 mol) Natriummethylat (als 30%ige Lösung in Methanol) bei Raumtemperatur versetzt und 30 min. bei dieser Temperatur gerührt. Anschließend wurden 200 g (1,328 mol) 3-Chlor-2,2-dimethylpropionsäuremethylester zugetropft und das Reaktionsgemisch 5 Stunden nachgerührt. Nach Zugabe von 95,2 g (1,76 mol) Natriummethylat (als 30%ige Lösung in Methanol) wurde weitere 15 Stunden nachgerührt. Danach wurde Lösungsmittel unter vermindertem Druck entfernt, der Rückstand mit 150 ml Wasser verrührt, die wäßrige Phase zweimal mit Dichlormethan extrahiert, mit 2 N Salzsäure angesäuert und erneut zweimal mit Dichlormethan extrahiert und aus der organischen Phase des 4,4-Dimethylisoxazolidin-3-on in 71%iger Ausbeute erhalten. Nach Verrühren mit Ether fiel die Verbindung kristallin mit einem Fp. von 72-74°C an.

Entsprechend Beispiel wurde die Umsetzung mit 3-Chlor-2,2-dimethylpropionsäureethylester in Gegenwart von zunächst Natriummethylat und anschließend Kaliumhydroxid als Base durchgeführt, wobei die o.g. Verbindung in 73%iger Ausbeute anfiel. Dieselbe Ausbeute wurde erhalten, wenn man das rohe Umsetzungsgemisch in der Weise aufarbeitete, daß man gasförmigen Chlorwasserstoff einleitete, den sich bildenden Niederschlag absaugte und das Filtrat einengte.

## Patentansprüche

1. Verfahren zur Herstellung von Isoxazolidin-3-onen der Formel I

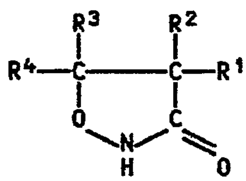

I,

in der $R^1$ und $R^2$ verzweigte oder unverzweigte Alkylreste mit 1 bis 8 Kohlenstoffatomen bedeuten, die unter den Reaktionsbedingungen inerte Substituenten tragen können und $R^3$ und $R^4$ Wasserstoff, Chlor, Brom oder einen aliphatischen Rest darstellen, oder von deren Salzen, durch Umsetzung von Hydroxylamin oder dessen Salzen mit einem Ester der Formel II

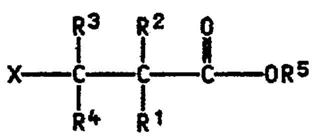

II,

in der X für Chlor oder Brom steht und $R^5$ einen aliphatischen, cycloaliphatischen, araliphatischen oder aromatischen Rest bedeutet, in Gegenwart einer Base und gegebenenfalls Freisetzen der Isoxazolidin-3-one aus deren Salzen durch Ansäuern des Reaktionsgemisches, dadurch gekennzeichnet, daß man die Umsetzung in einem inerten organischen Lösungsmittel oder einem wäßrig organischen Lösungsmittelgemisch ohne Isolierung von Zwischenstufen durchführt.

2. Verfahren nach Anspruch 1, dadurch gekennzeichnet, daß man 0,8 bis 1,5 mol Hydroxylamin je Mol Ausgangsstoff II verwendet.

3. Verfahren nach den Ansprüchen 1 und 2, dadurch gekennzeichnet, daß man als Lösungsmittel einen niedermolekularen Alkohol verwendet.

4. Verfahren nach den Ansprüchen 1 bis 3, dadurch gekennzeichnet, daß man als Base ein Alkalimetallalkoholat verwendet.

5. Verfahren nach den Ansprüchen 1 bis 4, dadurch gekennzeichnet, daß man 2 bis 6 mol Base je Mol Ausgangsstoff II verwendet.

## Claims

1. A process for preparing an isoxazolidin-3-one of the formula I

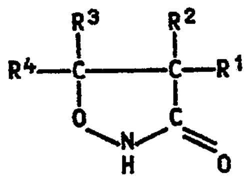

I,

where $R^1$ and $R^2$ are each branched or unbranched alkyl of 1 to 8 carbon atoms which may carry substituents which are inert under the reaction conditions, and $R^3$ and $R^4$ are each hydrogen, chlorine, bromine or an aliphatic radial, or a salt thereof, by reacting hydroxyl mine or a salt thereof with en ester of the formula II

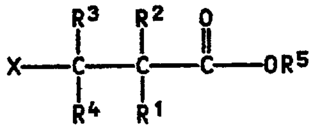

II,

where X is chlorine or bromine and $R^5$ is an aliphatic, cyloaliphatic, araliphatic or aromatic radical, in the presence of a base and, if desired, freeing the isoxazolidin-3-one from its salt by acidifying the reaction mixture, which comprises carrying out the reaction in an inert organic solvent or an aqueous organic solvent mixture without isolation of intermediates.

2. A process as claimed in claim 1, wherein from 0.8 to 1.5 moles of hydroxylamine are used per mole of starting material II.

3. A process as claimed in either of claims 1 and 2, wherein the solvent used is a low molecular weight alcohol.

4. A process as claimed in any of claims 1 to 3, wherein the base used is an alkali metal alcoholate.

5. A process as claimed in any of claims 1 to 4, wherein from 2 to 6 moles of base are used per mole of starting material II.

## Revendications

1. Procédé de préparation d'isoxazolidine-3-ones de formule I

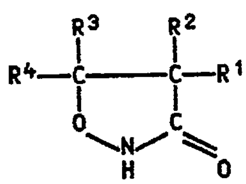

I,

dans laquelle $R^1$ et $R^2$ sont des restes alkyle ramifiés ou non ramifiés avec de 1 à 8 atomes de carbone, qui peuvent porter des substituants inertes dans les conditions de la réaction, et $R^3$ et $R^4$ représentent des atomes d'hydrogène, de chlore ou de brome ou un reste aliphatique, ou de leurs sels, par réaction d'hydroxylamine ou de ses sels avec un ester de formule II

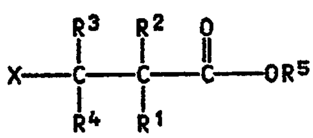

II,

dans laquelle X est le chlore ou le brome et $R^5$ un reste aliphatique, cycloaliphatique, araliphatique ou aromatique, en présence d'une base, et éventuellement libération de l'isoxazolidine-3-one de ses sels par acidification du mélange réactionnel, caractérisé en ce qu'on effectue la réaction dans un solvant organique inerte ou dans un mélange solvant organique/aqueux sans isolement des substances intermédiaires.

2. Procédé selon la revendication 1, caractérisé en ce qu'on utilise de 0,8 à 1,5 mole d'hydroxylamine par mole de substance de départ II.

3. Procédé selon les revendications 1 et 2, caractérisé en ce qu'on utilise comme solvant un alcool de faible poids moléculaire.

4. Procédé selon les revendications 1 à 3, caractérisé en ce qu'on utilise comme base un alcoolate de métal alcalin.

5. Procédé selon les revendications 1 à 4, caractérisé en ce qu'on utilise de 2 à 6 moles de base par mole de substance de départ II.